(19) Europäisches Patentamt
European Patent Office
Office européen des brevets

(11) **EP 3 672 533 B1**

(12) **EUROPÄISCHE PATENTSCHRIFT**

(45) Veröffentlichungstag und Bekanntmachung des Hinweises auf die Patenterteilung:
**24.02.2021  Patentblatt 2021/08**

(21) Anmeldenummer: **18789614.7**

(22) Anmeldetag: **18.10.2018**

(51) Int Cl.:
***A61F 2/30*** *(2006.01)*

(86) Internationale Anmeldenummer:
**PCT/EP2018/078555**

(87) Internationale Veröffentlichungsnummer:
**WO 2019/077045 (25.04.2019 Gazette 2019/17)**

(54) **IMPLANTAT**

IMPLANT

IMPLANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.10.2017   DE 102017124483**

(43) Veröffentlichungstag der Anmeldung:
**01.07.2020   Patentblatt 2020/27**

(73) Patentinhaber: **Friedrich-Alexander-Universität Erlangen-Nürnberg**
**91054 Erlangen (DE)**

(72) Erfinder:
• **GELSE, Kolja**
  **91052 Erlangen (DE)**
• **FEY, Tobias**
  **91058 Erlangen (DE)**
• **KRÖNKE, Gerhard**
  **91054 Erlangen (DE)**
• **KLEYER, Arnd**
  **91052 Erlangen (DE)**
• **HUEBER, Axel**
  **91052 Erlangen (DE)**

• **RENNER, Nina**
  **91052 Erlangen (DE)**
• **PACHOWSKY, Milena**
  **91052 Erlangen (DE)**
• **BIGGEMANN, Jonas**
  **91052 Erlangen (DE)**
• **SCHOLTYSEK, Carina**
  **90579 Langenzenn (DE)**
• **GREIL, Peter**
  **91085 Weisendorf (DE)**
• **SCHETT, Georg**
  **91054 Erlangen (DE)**
• **PEZOLDT, Marc**
  **99326 Ilmtal (DE)**
• **STUMPF, Martin**
  **91058 Erlangen (DE)**

(74) Vertreter: **Dr. Gassner & Partner mbB**
**Wetterkreuz 3**
**91058 Erlangen (DE)**

(56) Entgegenhaltungen:
**EP-A2- 1 712 205      WO-A1-2011/112145**
**WO-A1-2016/024248      US-A- 4 839 215**

EP 3 672 533 B1

**Beschreibung**

Die Erfindung betrifft ein Implantat zum Ersatz von Knochen- oder Knorpelmaterial. Das Implantat ist insbesondere zur Wiederherstellung funktioneller Gelenkoberflächen geeignet. Die Erfindung betrifft außerdem einen Kit.

Die WO 2014/006519 A1 offenbart ein dreidimensionales poröses Implantat, welches aus einer Vielzahl übereinander gestapelter Gelege gebildet ist. Die Gelege sind fest derart miteinander verbunden, dass zwischen benachbarten Gelegen Lücken oder Kanäle verbleiben. Das bekannte Implantat eignet sich insbesondere nicht zur Wiederherstellung von funktionellen Gelenkoberflächen.

Zur Wiederherstellung funktioneller Gelenkoberflächen sind nach dem Stand der Technik monolithische Implantate bekannt. Es wird dazu beispielhaft auf die folgenden Dokumente verwiesen: DE 103 03 660 B4, EP 0 144 209 B1, EP 0 197 441 B1, DE 100 22 260 C2, DE 101 57 315 C1, EP 1 646 334 B1, EP 1 442 726 B1, EP 2 104 471 B1, EP 2 296 583 B1.

Die nachveröffentlichte DE 10 2016 211 201 A1 offenbart ein flexibles Knochenimplantat, bei dem Strukturelemente einer ersten Gruppe mit Strukturelementen einer zweiten Gruppe lückenlos miteinander verbunden sind. Die Strukturelemente weisen eine unterschiedliche Härte auf.

Die EP 0 654 250 A offenbart ein Gitter-Implantat zur Überbrückung von Knochendefekten oder zur Fixierung von Knochenfragmenten.

Zur Wiederherstellung einer funktionellen Gelenkoberfläche mittels eines monolithischen Implantats ist es erforderlich, gesundes Gewebe, insbesondere die biomechanisch wichtige subchondrale Knochenplatte zu entfernen. Das ist aufwändig und für den Patienten belastend. Abgesehen davon können sich monolithische Implantate lockern.

Die EP 1 712 205 A2 offenbart ein Implantat zum Ersatz von Knochen- oder Knorpelmaterial, gebildet aus einer Vielzahl von aus einem nichtmetallischen, linear elastischen Material hergestellten Bausteinen, wobei ein Baustein mit benachbarten Bausteinen mittels eines Polymermaterials so verbunden ist, dass zwischen den benachbarten Bausteinen Lücken verbleiben und die benachbarten Bausteine relativ zueinander beweglich sind. Das Polymermaterial bildet eine Polymerschicht, welche die Bausteine überlagert und an die Oberseite der Bausteine angebunden ist.

Im Hinblick auf die Wiederherstellung funktioneller Gelenkoberflächen konzentriert sich die Forschung derzeit auf die Reparatur von Knorpelgewebe, beispielsweise durch autologe Chondrozytentransplantation, Mikrofrakturierung oder Matrix-induzierte Chondrogenese. Damit ist es möglich, Knorpelmaterial ausreichender Qualität herzustellen. Allerdings ist damit lediglich die Behandlung kleiner chondraler Läsionen mit noch intakten benachbarten Wirtsknorpel möglich. Eine Behandlung von großflächigen ostroarthritischen Läsionen ist damit nicht möglich.

Aufgabe der Erfindung ist es, die Nachteile nach dem Stand der Technik zu beseitigen. Es soll insbesondere ein Implantat angegeben werden, welches sich einfach applizieren lässt. Nach einem weiteren Ziel der Erfindung soll das Implantat sich zur Wiederherstellung funktioneller Gelenkoberflächen und Knochenmaterial eignen.

Diese Aufgabe wird durch die Merkmale des Anspruchs 1 gelöst. Zweckmäßige Ausgestaltungen der Erfindung ergeben sich aus den Merkmalen der abhängigen Patentansprüche. Gegenstand der Erfindung ist ferner ein Kit.

Nach Maßgabe der Erfindung wird ein Implantat zum Ersatz von Knochen- oder Knorpelmaterial vorgeschlagen, gebildet aus einer Vielzahl von aus einem nichtmetallischen, linear elastischen Material hergestellten Bausteinen, wobei ein Baustein mit benachbarten Bausteinen mittels eines viskoelastischen Polymermaterials so verbunden ist, dass zwischen den benachbarten Bausteinen Lücken verbleiben und die benachbarten Bausteine relativ zueinander beweglich sind.

Im Sinne der vorliegenden Erfindung wird unter dem Begriff "Lücke" ein Raum zwischen mehreren benachbarten und miteinander mittels des Polymermaterials verbundenen Bausteinen verstanden, der zumindest teilweise durch Flächen der Bausteine begrenzt ist. Die Flächen können zumindest abschnittsweise mit einem Material beschichtet sein, welches verschieden sein kann vom Polymermaterial und vom Material, aus dem die die Bausteine hergestellt sind. Im Raum kann ein Füllmaterial aufgenommen sein, welches verschieden ist vom Polymermaterial und vom Material, aus dem die Bausteine hergestellt sind.

Das vorgeschlagene Implantat ist in Abkehr vom Stand der Technik insgesamt ein hochflexibles Gebilde. Das Implantat passt sich wegen der viskoelastischen Eigenschaften der Verbindung der Bausteine an die Form einer Unterlage oder eines Hohlraums an. Es ist nicht oder nur in einem geringen Maß erforderlich, die Unterlage, beispielsweise eine subchondrale Knochenplatte, an die Form des Implantats anzupassen. Die aus dem viskoelastischen Material hergestellte Verbindung lässt sich leicht, beispielsweise mittels eines Messers, trennen. Infolgedessen ist es schnell und einfach möglich, das Implantat in seiner Form an die Form der Unterlage anzupassen. Das Implantat kann individuell an die Gegebenheiten des jeweiligen Patienten angepasst werden.

Zumindest ein Teil der aus dem nichtmetallischen, linear elastischen Material hergestellten Bausteine stellt eine hoch belastbare und zugleich reibungsarme Oberfläche bereit. Die zwischen den Bausteinen gebildeten Lücken ermöglichen nach der Applikation des Implantats eine Aufnahme von Zellmaterial, beispielsweise chondrogenen oder osteogenen Zellen, oder von bioaktivem Material. Das ermöglichte eine zelluläre Besiedelung des Implantats und damit eine lange Zeit haltbare Wiederherstellung beispielsweise einer Gelenkoberfläche.

Die Verbindung der Bausteine aus dem viskoelastischen Polymermaterial ermöglicht einen weitgehend formschlüssigen Kontakt der Bausteine mit einer Unterlage oder einem Hohlraum. Die Form des Implantats passt sich an die Form der Unterlage oder des Hohlraums an. Infolgedessen kann bei Verwendung des erfindungsgemäßen Implantats wertvolles, gesundes Zellmaterial, insbesondere Knochengewebe, weitgehend erhalten werden.

Das vorgeschlagene Implantat ist aus einer Vielzahl von Bausteinen gebildet. Die Bausteine können mehrere aus unterschiedlichen Materialien hergestellte Bausteine umfassen. Damit ist es möglich, das Implantat beispielsweise in unterschiedliche funktionelle Zonen zu gliedern. Es können beispielsweise Bausteine aus einem nichtresorbierbaren keramischen Material mit Bausteinen aus einem resorbierbaren, bioaktiven keramischen Material kombiniert werden.

Nach einer weiteren vorteilhaften Ausgestaltung weist das linear elastische Material einen E-Modul von zumindest 10 GPa auf. Das linear elastische Material ist im Wesentlichen spröde. Es zeichnet sich üblicherweise durch eine hohe Härte aus. Das linear elastische Material kann auch eine offene Porosität aufweisen. Ein kommunizierender Porenraum hat dabei üblicherweise ein Porenvolumen im Bereich von 10 bis 80 Vol.%, vorzugsweise 20 bis 70 Vol.%, besonders bevorzugt 30 bis 60 Vol.%.

Unter einem Polymermaterial mit "viskoelastischen Eigenschaften" wird ein Polymermaterial verstanden, welches bei Raumtemperatur eine Bruchdehnung von zumindest 0,5%, vorzugsweise zumindest 2%, besonders bevorzugt zumindest 5%, aufweist.

Als linear elastische Materialien können verwendet werden Metalle, nichtmetallische anorganische Werkstoffe sowie Komposite, welche Polymermaterialien enthalten können.

Vorteilhafterweise ist das linear elastische Material aus der folgenden Gruppe ausgewählt: Keramik, Glaskeramik, Glas, oder ein zumindest eines der vorgenannten Materialien enthaltender Komposit-Werkstoff. Bei dem Komposit-Werkstoff ist die Matrix vorteilhafterweise aus einem Polymermaterial gebildet.

Das linear elastische Material ist insbesondere aus der folgenden Gruppe ausgewählt: Aluminiumoxid, Hydroxylapatit, Beta-Tricalciumphosphat (TCP), $BaTiO_3$-Epoxidharz-Komposit, Bioglas, Bioglas-Epoxidharz-Komposite, bleifreie Epoxidharz-Komposite, bleifreie Keramiken, z. B. Lithium-, Natrium-, Kalium-Niobat, Keramik/präkeramische Polymere-Komposite, beispielsweise Polysiloxane, Polysilazane, Polyphosphazene, vernetzte präkeramische Polymere, gesinterte präkeramische Polymere. Die präkeramischen Polymere können mit einem Füllstoff gefüllt sein, wobei ein Anteil des Füllstoffs zumindest 5 Vol.%, vorzugsweise zumindest 20 Vol.%, besonders bevorzugt zumindest 30 Vol.% beträgt.

Nach einer weiteren Ausgestaltung ist zumindest ein Teil der Bausteine aus mehreren Schichten gebildet, welche aus einem unterschiedlichen Material hergestellt sind. Damit ist es möglich, eine dem Knochen zugewandte Fläche der Bausteine beispielsweise eine Funktion zu verleihen, welche eine Anbindung an den Knochen unterstützt. Eine dem Knochen abgewandte Seite der Bausteine kann z. B. aus einer Schicht mit tribologisch vorteilhaften Eigenschaften gebildet sein. Aus mehreren Schichten hergestellte Bausteine können beispielsweise mittels Folientechnik, Niederdruckspritzguss, 3D-Druck, Kalt-/Warm-Kompaktiertechniken und dgl. hergestellt werden.

Nach einer weiteren Ausgestaltung umfasst zumindest ein Teil der Bausteine eine Ober- und Unterseite sowie die Ober- mit der Unterseite verbindende Seitenflächen. Die Bausteine können in Draufsicht auf die Oberseite einen polygonalen Umriss mit zumindest m Ecken aufweisen, wobei m eine natürliche Zahl ≥ 3 ist. Die Bausteine können insbesondere nach Art eines Prismas oder Pyramidenstumpfs ausgebildet sein. Zweckmäßigerweise weisen die Bausteine eine n-zählige Symmetrieachse auf, wobei für n gilt:

$$n = m \,/\, a,$$

wobei a eine natürliche Zahl ist. D. h. bei den Bausteinen kann es sich beispielsweise um ein Prisma mit einer drei- oder mehrzähligen Symmetrieachse handeln. Auch andere geometrische Formen sind möglich, beispielsweise kann ein Baustein nach Art eines Kreuzes mit acht Ecken ausgebildet sein. In diesem Fall kann das Kreuz eine vier-zählige Symmetrieachse aufweisen.

Des Weiteren kann zumindest ein Teil der Bausteine eine ring- oder rohrartige Geometrie aufweisen. Derartige Geometrien eignen sich insbesondere zur Durchführung von Befestigungsmitteln, beispielsweise Nägeln oder Schrauben.

Vorteilhafterweise weist ein Übergang zwischen der Oberseite und den Seitenflächen eine Rundung auf. Eine solche Rundung verleiht der aus der Vielzahl von Bausteinen gebildeten Gesamt-Oberseite tribologisch verbesserte Eigenschaften. Eine Reibung an Kanten im Übergang zwischen der Oberseite und den Seitenflächen wird vermieden.

Nach einer weiteren Ausgestaltung ist die Ober- und/oder Unterseite mit einem vorgegebenen Radius gekrümmt. Ein solcher Radius kann vor der Herstellung des Implantats durch eine röntgenographische 3D-Modellierung des Knochens ermittelt werden. Damit kann ein verbesserter, formschlüssiger Kontakt zwischen dem Implantat und der Unterlage erreicht werden. D. h. der vorgegebene Radius ist an die Geometrie des Gelenks angepasst.

Zweckmäßigerweise weist die Oberseite eine erste Rauheit und die Unterseite eine zweite Rauheit auf, wobei die erste Rauheit kleiner als die zweite Rauheit ist. Unter "Rauheit" wird im Sinne der vorliegenden Erfindung die "mittlere Rauheit", dargestellt durch das Symbol $R_a$, verstanden. Sie gibt den mittleren Abstand eines Messpunkts auf der Oberfläche zur Mittellinie an. Die Mittellinie schneidet innerhalb einer Bezugsstrecke das wirkliche Profil so, dass die Summe der Profilabweichungen bezogen auf die Mittellinie minimal wird. Die mittlere Rauheit $R_a$ entspricht also dem arithmetischen Mittel der betragsmäßigen Abweichung von der Mittellinie. In zwei Dimensionen berechnet sie sich aus:

$$R_a = \frac{1}{MN} \sum_{m=1}^{M} \sum_{n=1}^{N} |z(x_m, y_n) - \langle z \rangle|$$

wobei der Mittelwert durch

$$\langle z \rangle = \frac{1}{MN} \sum_{m=1}^{M} \sum_{n=1}^{N} z(x_m, y_n)$$

gegeben ist.

Bei Bausteinen mit einer ring- oder rohrartigen Geometrie können an einem Außenumfang mehrere Vorsprünge zum Anbinden des Polymermaterials angeformt sein. Die Vorsprünge können sich in Axialrichtung am Außenumfang erstrecken und beispielsweise eine Satteldach-artige Form aufweisen. Sie können gleichmäßig um den Außenumfang verteilt sein. Vorteilhafterweise sind zumindest drei derartige Vorsprünge am Außenumfang vorgesehen.

Das Polymermaterial hat in Abgrenzung zum linear elastischen Material einen E-Modul von weniger als 10 GPa. Das Polymermaterial ist erfindungsgemäß aus der folgenden Gruppe ausgewählt: Epoxidharz, präkeramische Polymere, Silikongummi, Polylactid (PDLA, PLLA), Polycaprolacton, Polymethylmethaacrylate, Polylactid-co-Glycolid (PLGA), Polyhydroxyalkanoate (PHBHHX), Fibrin, Butyrate, Seide, Chitosan. Bei dem Polymermaterial kann es sich insbesondere um ein biokompatibles Polymer handeln, welches resorbierbar oder nicht resorbierbar ist.

Nach einer weiteren vorteilhaften Ausgestaltung umfassen die Bausteine mehrere Teilmengen, wobei Bausteine einer Teilmenge sich von Bausteinen einer anderen Teilmenge in ihrer Geometrie unterscheiden. Beispielsweise können Bausteine mit einer prismatischen Geometrie mit Bausteinen mit einer ring- oder rohrförmigen Geometrie kombiniert werden.

Erfindungsgemäß bildet das viskoelastische Polymermaterial eine Polymerschicht, welche die Bausteine überlagert und an die Oberseite der Bausteine angebunden ist. Die Polymerschicht weist zweckmäßigerweise eine Dicke im Bereich von 50 bis 1500 μm auf. Die Polymerschicht weist erfindungsgemäß Durchbrüche auf. Sie kann netz- oder gitterartig ausgebildet sein.

Die vorgeschlagene Polymerschicht erfüllt vorteilhafterweise zwei Funktionen. Sie dient einerseits zur flexiblen Verbindung der Bausteine. Andererseits stellt die Polymerschicht eine glatte Oberfläche bereit, welche im applizierten Zustand des Implantats im Kontakt mit der anderen Gelenkhälfte ist. Damit kann ein unerwünschter unmittelbarer Kontakt der anderen Gelenkhälfte mit Ecken oder Kanten der Bausteine vermieden werden. Eine Schädigung der anderen Gelenkhälfte kann vermieden werden. - Die Polymerschicht bildet insbesondere eine Reibfläche mit knorpelähnlichen Eigenschaften. Damit kann die Oberflächenrauigkeit der Bausteine vermindert und/oder Höhenunterschiede zwischen den Bausteinen ausgeglichen werden. Ein Kontakt zwischen den Gelenkhälften kann auch durch das Einlegen einer Gleitschicht, z. B. einer Proteinlage, vermieden werden.

Nach einer weiteren Ausgestaltung ist ein Baustein mit benachbarten Bausteinen über zumindest drei aus dem viskoelastischen Polymermaterial hergestellte Brücken verbunden. Die Brücken sind Verbindungen, welche zweckmäßigerweise an den Seitenflächen, insbesondere an Vorsprüngen oder Kanten der Seitenflächen, angebunden sind.

Nach einer weiteren, besonders vorteilhaften Ausgestaltung ist die Oberseite der Bausteine mit dem Polymermaterial oder einem weiteren Polymermaterial beschichtet. Die aus dem Polymermaterial oder dem weiteren Polymermaterial gebildete Beschichtung kann zumindest abschnittsweise mit der Polymerschicht verbunden sein.

Das Implantat eignet sich insbesondere zur Wiederherstellung einer funktionellen Gelenkoberfläche. Nach einer weiteren besonders vorteilhaften Ausgestaltung ist eine einzige Lage von in einer Ebene angeordneten Bausteinen mittels des Polymermaterials zu einer flexiblen Schicht verbunden.

Nach einer weiteren Ausgestaltung sind mehrere aufeinander gestapelte Lagen von Bausteinen zu einer flexiblen Schicht oder einem flexiblen Block verbunden. In der Ausgestaltung einer flexiblen Schicht eignet sich das Implantat wiederum zur Herstellung von funktionellen Gelenkflächen. In der Ausgestaltung eines flexiblen Blocks eignet sich das Implantat zum Füllen von Hohlräumen in Knochen oder allgemein zum Modellieren von Knochenmaterial. Bei übereinander gestapelten Lagen von Bausteinen können die Bausteine beispielsweise aus Pyramiden mit einer drei- oder mehreckigen Grundfläche gebildet sein. Insbesondere nach Art eines Tetraeders ausgebildete Pyramiden eignen sich zur Herstellung flexibler dreidimensionaler Implantate. Dabei ist jeder pyramidale Baustein über seine Ecken mit benachbarten pyramidalen Bausteinen über Brücken aus dem viskoelastischen Polymermaterial verbunden.

Die Erfindung betrifft ferner einen Kit, umfassend ein erfindungsgemäßes Implantat und Befestigungsmittel zum Befestigen des Implantats. Bei den Befestigungsmitteln kann es sich beispielsweise um Nägel, Schrauben und dgl. handeln. Die Befestigungsmittel können aus dem zur Herstellung der Bausteine verwendeten Material, aus Metall oder einer geeigneten Keramik hergestellt sein. Die Befestigungsmittel werden vorzugsweise durch in den Bausteinen vorgesehene Durchbrüche hindurchgeführt. Insbesondere eignen sich zum Durchführen der Befestigungsmittel ring- oder rohrartige Bausteine.

Nachfolgend werden Ausführungsbeispiele der Erfindung anhand der Zeichnungen näher erläutert. Es zeigen:

Fig. 1a     Eine perspektivische Ansicht eines ersten Bausteins,
Fig. 1b     eine Draufsicht auf den Baustein gemäß Fig. 1a,
Fig. 2a     eine perspektivische Ansicht eines zweiten Bausteins
Fig. 2b     eine Draufsicht auf den Baustein gemäß Fig. 2a,
Fig. 3a     eine perspektivische Ansicht eines dritten Bausteins,
Fig. 3b     eine Draufsicht auf den Baustein gemäß Fig. 3a,
Fig. 4a     eine perspektivische Ansicht eines vierten Bausteins,
Fig. 4b     eine Draufsicht auf den Baustein gemäß Fig. 4a,
Fig. 5      eine Draufsicht auf eine Variante des zweiten Bausteins,
Fig. 6      eine perspektivische Ansicht einer Variante des ersten Bausteins,
Fig. 6a     eine schematische Ansicht zweier Bausteine mit einer dazwischen befindlichen Lücke,
Fig. 7      eine perspektivische Ansicht eines ersten Implantats,
Fig. 8      eine perspektivische Ansicht eines zweiten Implantats,
Fig. 9      eine perspektivische Ansicht eines dritten Implantats,
Fig. 10     eine perspektivische Ansicht eines vierten Implantats,
Fig. 11     eine perspektivische Ansicht des vierten Implantats in auf einem Knochen applizierter Form,
Fig. 12     eine schematische Ansicht eines fünften Implantats,
Fig. 13     eine schematische Ansicht von Bausteinen mit daran angebundener Polymerschicht,
Fig. 13a    eine schematische Ansicht weiterer Bausteine mit einer Beschichtung,
Fig. 14     eine schematische Ansicht weiterer Bausteine mit daran angebundener Zwischen- und Polymerschicht,
Fig. 15a    eine schematische Ansicht eines sechsten Implantats,
Fig. 15b    eine schematische Ansicht eines siebten Implantats,
Fig. 15c    eine schematische Ansicht eines achten Implantats,
Fig. 16     eine schematische Draufsicht auf ein neuntes Implantat,
Fig. 17     eine schematische Draufsicht auf ein zehntes Implantat und
Fig. 18     eine perspektivische Ansicht eines siebten Bausteins.

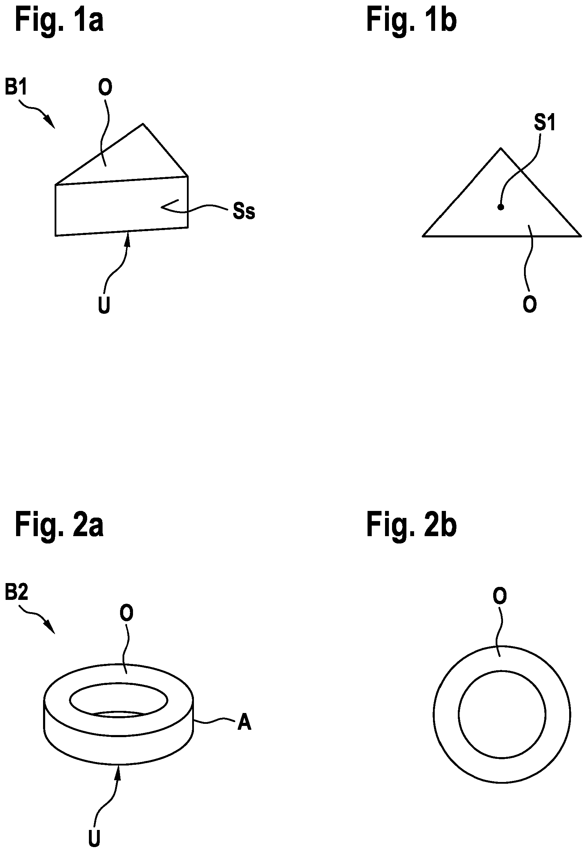

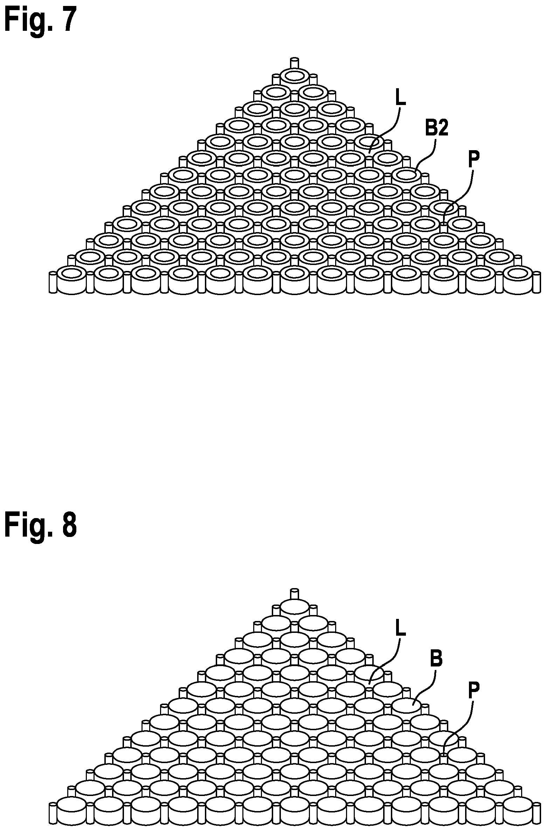

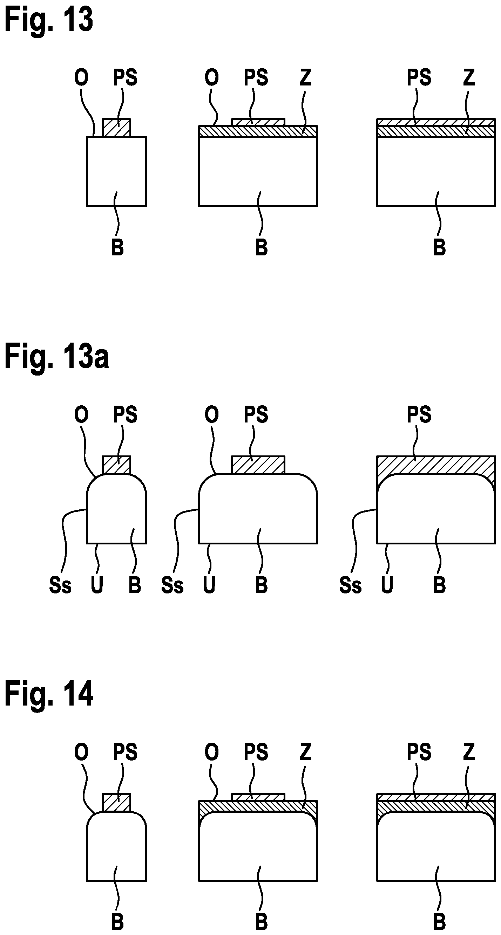

Die Fig. 1 bis 6 und 18 zeigen Beispiele von Bausteinen B, aus denen erfindungsgemäße Implantate hergestellt werden können. Ein maximaler Durchmesser der Bausteine B kann 0,3 bis 10,0 mm, vorzugsweise 0,5 bis 7,0 mm, besonders bevorzugt 2,0 bis 6,0 mm, betragen. Zur Herstellung einer ersten Variante eines Implantats, welches beispielhaft in den Fig. 7 bis 12 wiedergegeben ist, werden die Bausteine B mittels flexibler Brücken P zu einem flexiblen Gebilde verbunden. Bei der in den Fig. 14 bis 17 gezeigten zweiten Variante sind die Bausteine B mittels einer Polymerschicht, welche als Polymergitter ausgebildet sein kann, zu einem flexiblen Gebilde verbunden.

Die nachfolgenden Tabellen geben beispielhaft geeignete Materialien zur Herstellung der Bausteine B sowie geeignete Polymermaterialien wieder:

Tabelle 1: Material für Bausteine

| Material | E-Modul [GPa] | KIC [MPa / m$^{1/2}$] | Verhalten |
|---|---|---|---|
| Aluminiumoxid $Al_2O_3$ | 385 | 3,6-4,4 | Linear-elastisch |
| Hydroxylapatit | 80-120 | 0,6-1,0 | Linear-elastisch |
| Beta-TCP | 21 | 2,3 | Linear-elastisch |
| Bioglas | 35 | 2 | Linear-elastisch |
| Poröses $Al_2O_3$ (30-70% Porosität) | 60-200 | | Linear-elastisch |

(fortgesetzt)

| Material | E-Modul [GPa] | KIC [MPa / m$^{1/2}$] | Verhalten |
|---|---|---|---|
| BaTiO$_3$ - Epoxydharz Komposite (5-45 Vol % BaTiO3) | 12-30 | | Linear-elastisch |
| LNKN - Epoxyharz Komposite (5-45 Vol % LNKN) | 12-30 | | Linear-elastisch |
| Kortikaler Knochen | 7-30 | 2-12 | |

Tabelle 2: Polymermaterialien

| Material | E-Modul [GPa] | KIC [MPa / m$^{1/2}$] | Bruchdehnung [%] | Verhalten |
|---|---|---|---|---|
| Epoxidharz | 4-8 | | 0.5-6, Epicure 9.4 | Visco-elastisch |
| Silikongummi | 0,045 | 0,03 | 2-100 | Visco-elastisch |
| Kollagen | 0,3-2,5 | 1-10 | 10-30 | Visco-elastisch |
| Polylactide | 2,3-3,5 | | 2-6, 5,3 | Visco-elastisch |
| Polycaprolactone | | | 1-200, teilweise 660 | Visco-elastisch |
| Polymethylmethaacrylate | 1,8-3,3 | | 1-100, Vitralit 4731 (328) | Visco-elastisch |
| Poly(lactic-co-glycolic acid) (PLGA) | | | 2-8 | Visco-elastisch |
| Polyhydroxyalkanoate (Hexanoate) (PHBHHx) | | | 8-15 | Visco-elastisch |
| Fibrin | | | | |
| Butyrate | | | | |
| Hyaluronsäure | 0,1-0,4 | | | |
| Seide | 0,01-0,4 | | | |
| Chitosan | 0,8-1,2 | | | |
| Alginat | 14 kPa | | | |

Die Fig. 1a und 1b zeigen einen ersten Baustein B1, welcher nach Art eines trigonalen Prismas ausgestaltet ist. Eine Oberseite des ersten Bausteins B1 ist mit dem Bezugszeichen O eine Unterseite mit dem Bezugszeichen U und die Ober- O mit der Unterseite U verbindenden Seitenflächen mit dem Bezugszeichen Ss bezeichnet. Die Grundfläche des ersten Baustein B1 ist aus einem gleichseitigen Dreieck gebildet. Der erste Baustein B1 weist eine drei-zählige erste Symmetrieachse S1 auf.

Fig. 2a und 2b zeigen einen zweiten Baustein B2. Der zweite Baustein B2 ist nach Art eines Rohrabschnitts ausgebildet. Ein Außenumfang ist mit dem Bezugszeichen A bezeichnet.

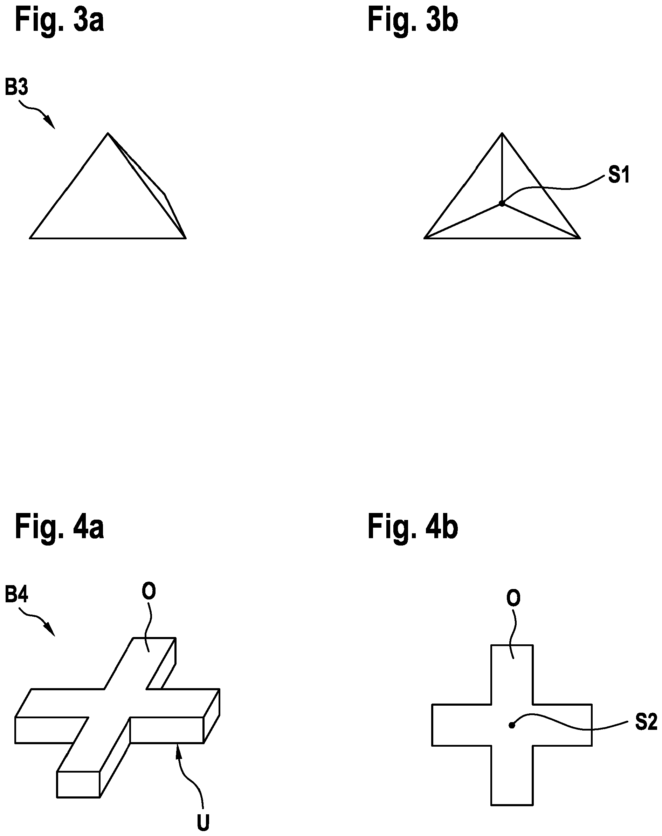

Die Fig. 3a und 3b zeigen einen dritten Baustein B3, welcher nach Art eines Tetraeders ausgebildet ist. Der Tetraeder weist als Grundfläche ein gleichseitiges Dreieck in einer drei-zähligen ersten Symmetrieachse S1 auf.

Die Fig. 4a und 4b zeigen einen vierten Baustein B4. Der vierte Baustein B4 ist nach Art eines Kreuzes ausgebildet. Er weist in der Draufsicht acht Ecken und eine vier-zählige zweite Symmetrieachse S2 auf.

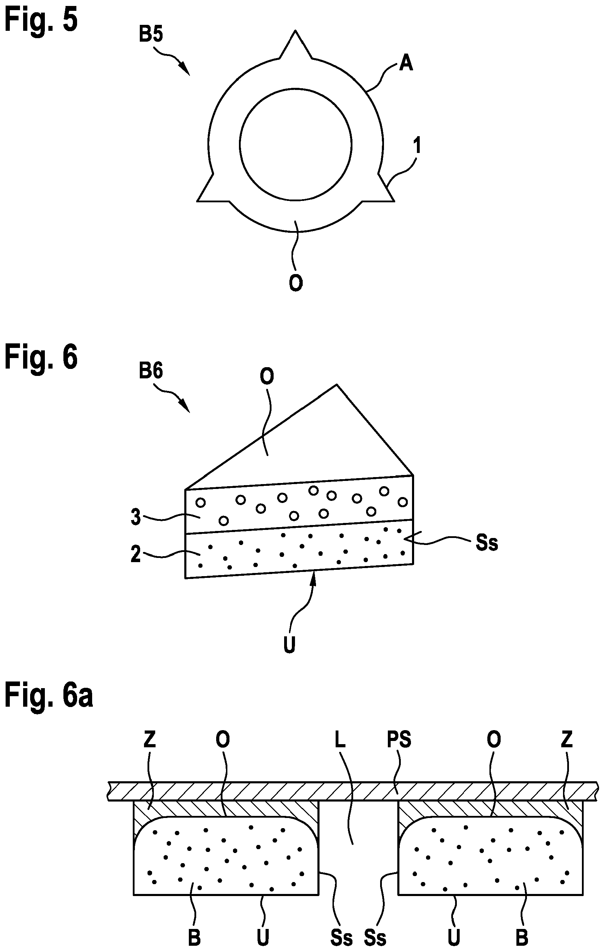

Fig. 5 zeigt als fünften Baustein B5 eine Variante des zweiten Bausteins B2. Dabei sind an einem Außenumfang A Vorsprünge 1 angeformt. Die Vorsprünge 1 sind gleichmäßig über den Außenumfang A verteilt.

Fig. 6 zeigt als sechsten Baustein B6 eine Variante des ersten Bausteins B1. Der sechste Baustein B6 ist aus einer ersten Schicht 2 und einer darüberliegenden zweiten Schicht 3 gebildet. Die erste Schicht 2, welche die Unterseite U umfasst, ist beispielsweise aus einem Material hergestellt, welches eine Anbindung an eine Unterlage, beispielsweise ein Knochen- oder Knorpelgewebe, unterstützt. Die zweite Schicht 3 ist auf einem davon verschiedenen Material hergestellt. Es kann sich dabei beispielsweise um ein tribologisch belastbares Material, beispielsweise Aluminiumoxid, ein bioaktives Material oder dgl. handeln. Die zweite Schicht 3 kann auch aus einer Abfolge mehrerer zweiter Schichten gebildet sein. Die Schichten können aus dem linear-elastischen Material und/oder dem viskoelastischen Material oder einer Kombination beider Materialien gebildet sein. - Die Oberseite O kann mit einer Beschichtung Z versehen sein, welche aus dem Polymermaterial und/oder einem weiteren Polymermaterial hergestellt ist, welches vom jeweils verwendeten Polymermaterial verschieden ist (hier nicht gezeigt).

Fig. 6a zeigt eine schematische Schnittansicht durch zwei Bausteine B mit einer dazwischen befindlichen Lücke L. Die Lücke L ist durch die Seitenflächen Ss der Bausteine B begrenzt. Bei der in Fig. 6a gezeigten Ausgestaltung sind die Kanten, welche die Oberseite O der Bausteine B begrenzen abgerundet. Auf der Oberseite O sowie einem Abschnitt der Seitenflächen Ss ist die Beschichtung Z vorgesehen.

Bei der hier gezeigten Ausgestaltung sind die Lücken L also abschnittsweise auch durch die Beschichtung Z begrenzt. Es kann auch sein, dass die Seitenflächen Ss vollständig mit der Beschichtung Z versehen sind. In diesem Fall werden die Lücken L durch die mit der Beschichtung Z versehenen Seitenflächen Ss begrenzt. In der Lücke L kann ein Füllmaterial (hier nicht gezeigt) aufgenommen sein. Bei dem Füllmaterial kann es sich um Zellen, ein Zell-Matrix-Konstrukt, bioaktives Material, z. B. verkapselte Zellen, Wachstums- und/oder Differenzierungsfaktoren oder dgl. handeln.

Die Fig. 7 bis 10 zeigen beispielsweise Implantate zur Wiederherstellung funktioneller Gelenkflächen. Die gezeigten Implantate sind jeweils aus in einer einzigen Ebene angeordneten Bausteinen B gebildet, welche mittels mehrerer Brücken P flexibel miteinander verbunden sind. Mit dem Bezugszeichen L sind Lücken zwischen den Bausteinen B bezeichnet. Die Lücken L dienen im applizierten Zustand der Aufnahme von Zellmaterial oder bioaktivem Material.

Bei dem in Fig. 7 gezeigten ersten Implantat entsprechen die Bausteine B dem in Fig. 2a und 2b gezeigten zweiten Baustein B2. Jeder Baustein B ist über drei Brücken P mit benachbarten Bausteinen B verbunden.

Bei dem in Fig. 8 gezeigten zweiten Implantat sind die Bausteine B zylindrisch ausgebildet. Auch hier ist jeder Baustein B über drei Brücken P mit benachbarten Bausteinen B verbunden.

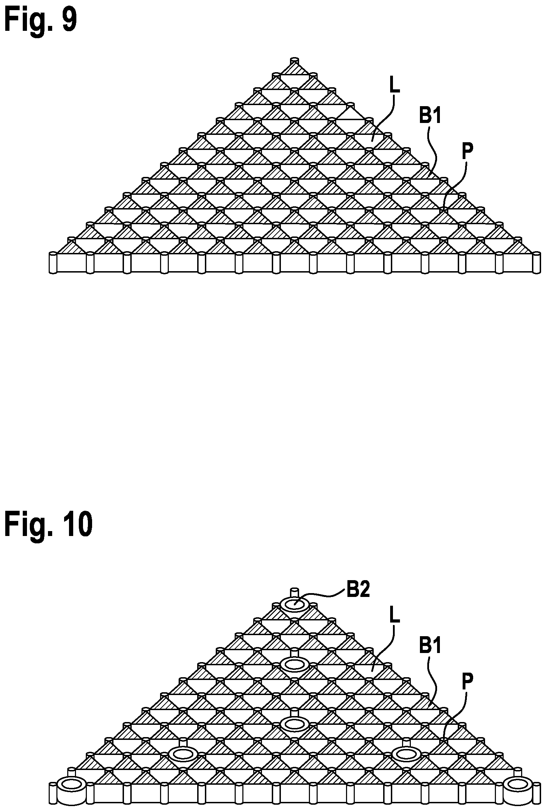

Bei dem in Fig. 9 gezeigten dritten Implantat sind die Bausteine B entsprechend dem in den Fig. 1a und 1b gezeigten ersten Baustein B1 ausgebildet. Auch hier sind die benachbarten Bausteine B jeweils über drei Brücken P miteinander verbunden. Die Verbindungen bzw. Brücken P sind jeweils an den etwa senkrecht zur Oberseite O verlaufenden Kanten der Bausteine B angebunden.

Bei dem in Fig. 10 gezeigten vierten Implantat sind Bausteine B unterschiedlicher Geometrien miteinander kombiniert. Das vierte Implantat umfasst erste B1 und zweite Bausteine B2. Die ersten B1 und die zweiten Bausteine B2 sind jeweils wiederum mittels dreier Brücken P miteinander flexibel verbunden. Die zweiten Bausteine B2 dienen der Durchführung von Befestigungsmitteln, beispielsweise Schrauben oder Nägeln 4.

Bei den in den Fig. 7 bis 10 gezeigten Ausführungsbeispielen weisen die Brücken P eine geometrisch definierte, nämlich eine zylindrische, Form auf. - Die Verbindungen bzw. Brücken P können aber auch in ihrer Form geometrisch voneinander verschieden sein.

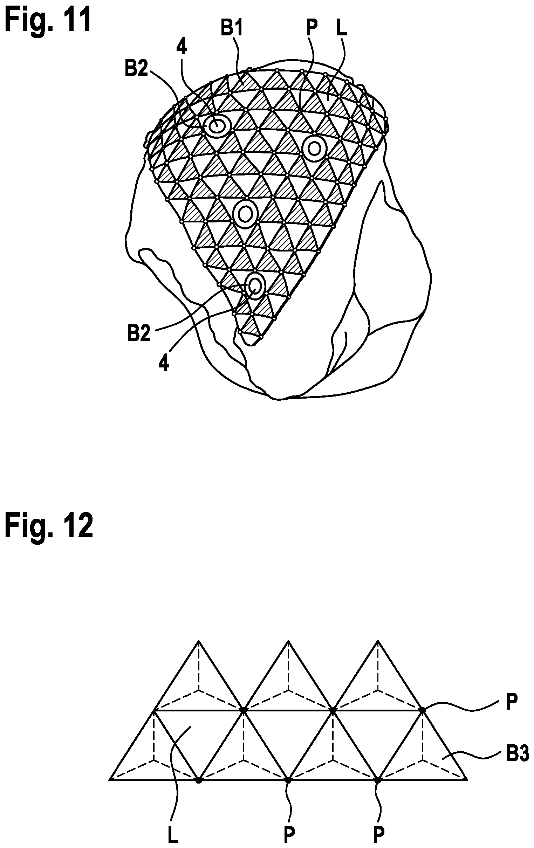

Fig. 11 zeigt das vierte Implantat gemäß Fig. 10 in auf einen Knochen applizierter Form. Dabei durchgreifen Befestigungsmittel, z. B. Nägel 4, die zweiten Bausteine B2 und verankern das vierte Implantat im Knochen. Es kann auch sein, dass ein zu Befestigungszwecken vorgesehener Stift einstückig mit einem Baustein verbunden ist und sich z. B. von dessen Unterseite erstreckt.

Fig. 12 zeigt schematisch ein fünftes Implantat, welches aus übereinander gelagerten Schichten gebildet ist. Jede Schicht ist aus flexibel mittels Brücken P miteinander verbundener dritter Bausteine B3 gebildet. Die Schichten sind wiederum mittels Brücken P miteinander verbunden. Statt der aus Tetraedern gebildeten dritten Bausteine können in ähnlicher Weise auch bipyramidal geformte Bausteine, beispielsweise trigonal oder tetragonal bipyramidale Bausteine, verwendet werden.

Fig. 13 zeigt schematisch Bausteine B, an deren Oberseite O jeweils eine Polymerschicht PS angebunden ist. Die Oberseite O kann mit einer Beschichtung Z versehen sein, welche aus dem Polymermaterial und/oder einem weiteren Polymermaterial hergestellt ist, welches vom jeweils verwendeten Polymermaterial verschieden ist. In diesem Fall ist die Beschichtung Z zwischen dem Baustein und der Polymerschicht PS angeordnet. Das weitere Polymermaterial kann aus den für das Polymermaterial angegebenen Materialien ausgewählt sein. Die Polymerschicht PS kann die Oberseite O abschnittsweise oder vollflächig überdecken.

Fig. 13a zeigt schematisch Bausteine B, deren an die Oberseite O angrenzenden Kanten abgerundet sind.

Fig. 14 zeigt weitere Bausteine B, an deren Oberseiten jeweils die Polymerschicht PS angebunden ist. Die an die Oberseite O angrenzenden Kanten der weiteren Bausteine B sind hier gerundet. Die auf der Oberseite O vorgesehene Beschichtung Z kann die Oberseite O abschnittsweise oder vollflächig überdecken. Die Beschichtung Z kann sich auch über die abgerundeten Kanten hinweg bis zu den Seitenflächen erstrecken. Sie kann auch die Seitenflächen zumindest abschnittsweise überdecken.

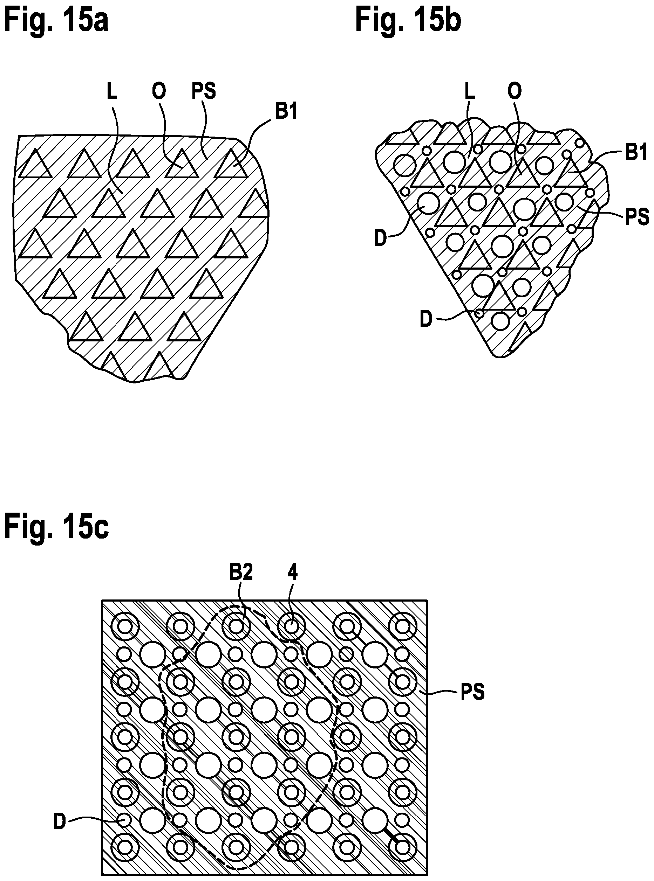

Fig. 15a zeigt eine schematische Ansicht eines sechsten Implantats. Bei dem sechsten Implantat ist die Polymerschicht PS durchgehend, d. h. ohne Durchbrüche, ausgebildet. Sie ist an die Oberseite der ersten Bausteine B1 angebunden. Die ersten Bausteine B1 sind regelmäßig angeordnet.

Bei dem in Fig. 15b gezeigten siebten Implantat weist die Polymerschicht PS regelmäßig angeordnete Durchbrüche D auf. Die Durchbrüche D befinden sich jeweils oberhalb der Lücken L zwischen den ersten Bausteinen B1.

Fig. 15c zeigt schematisch ein achtes Implantat. Das achte Implantat ist gebildet aus zweiten Bausteinen B2, welche vorzugsweise regelmäßig angeordnet sind. Die zweiten Bausteine B2 können an ihrer Oberseite O abgerundet sein. Die Polymerschicht PS ist an die Oberseite O oder eine auf der Oberseite O aufgebrachte Zwischenschicht Z (hier nicht gezeigt) angebunden. Die Polymerschicht PS weist, vorzugsweise regelmäßig angeordnete, runde Durchbrüche auf. - Das gezeigte achte Implantat kann ein Halbzeug bilden. Der Operateur kann nach Bedarf ein geeignetes Stück aus dem achten Implantat herausschneiden. Eine entsprechende Schnittlinie ist beispielhaft mit der unterbrochenen Linie gezeigt. Die zweiten Bausteine B2 eignen sich universell zur Durchführung von Befestigungsmitteln, beispielsweise Nägeln 4, Schrauben und dgl. Wegen der Verwendung der zweiten Bausteine B2 hat der Operateur besonders viele Freiheiten im Hinblick auf die Anbringung von Befestigungsmitteln.

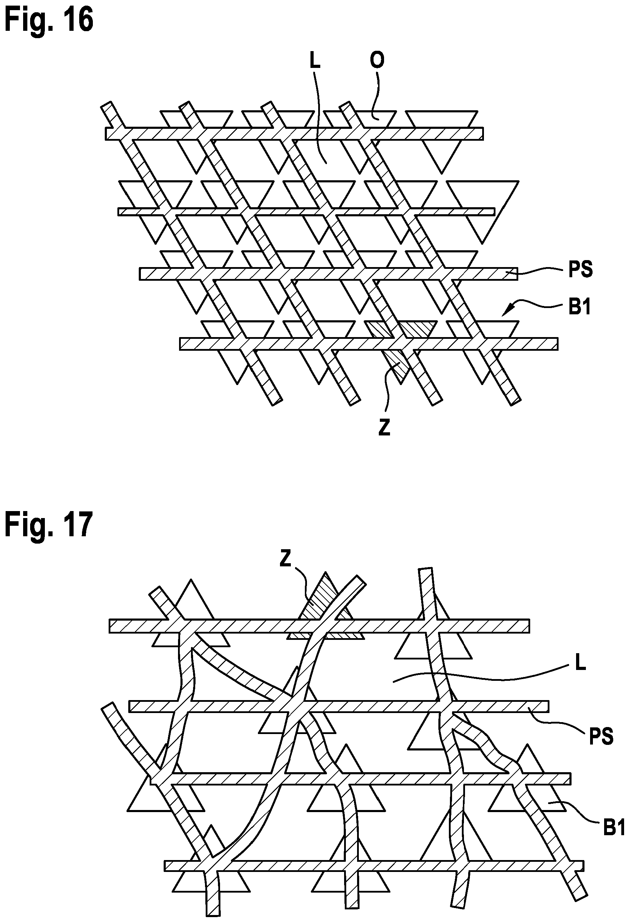

Bei dem in Fig. 16 schematisch gezeigten neunten Implantat sind erste Bausteine B1 ebenfalls regelmäßig angeordnet. Die Polymerschicht PS besteht hier aus einem regelmäßig gestalteten Gitter. Wie aus Fig. 16 ersichtlich ist, können die Oberflächen O der ersten Bausteine B1 vollständig oder auch nur abschnittsweise mit der Polymerschicht PS überlagert sein. Ein Teil der ersten Bausteine B1 kann mit der Beschichtung Z versehen sein.

Bei dem in Fig. 17 gezeigten zehnten Implantat sind die ersten Bausteine B1 unregelmäßig angeordnet. Die Polymerschicht PS ist aus einem unregelmäßig gestalteten Gitter gebildet. Ein Teil der ersten Bausteine B1 kann mit der Beschichtung Z versehen sein.

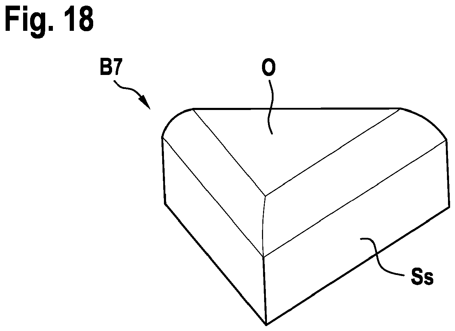

Fig. 18 zeigt eine perspektivische Ansicht eines siebten Bausteins B7. Der siebte Baustein B7 ist ähnlich dem ersten Baustein B1 ausgebildet, wobei hier jedoch die an die Oberseite O angrenzenden Kanten abgerundet sind.

Die Polymerschicht PS kann als Polymerfilm vorliegen. Die Polymerschicht PS kann mittels eines thermisch aktivierten Pressvorgangs mit beschichteten oder unbeschichteten Bausteinen B verbunden werden. Die Polymerschicht PS kann aber auch im 3D-Direktdruck, z. B. im FDM-Verfahren, auf beschichteten oder unbeschichteten Bausteinen B hergestellt werden. Insbesondere unregelmäßig gestaltete Gitter ermöglichen die Einstellung einer unterschiedlichen Flexibilität des Implantats. Die Flexibilität kann aber auch durch eine unterschiedliche Dicke in der Polymerschicht PS und/oder die Größe der Verbindungsfläche zwischen den Bausteinen B und der Polymerschicht PS und/oder die Größe und Anzahl der Durchbrüche variiert werden.

Insbesondere bei aus einer einzigen Lage von Bausteinen B gebildeten Implantaten bilden die Lücken L im auf eine Unterlage applizierten Zustand Hohlräume. Solche Hohlräume können mit einer zellbeladenen oder zellfreien Matrix verfüllt werden. Die Matrix kann Wachstums- und Differenzierungsfaktoren enthalten, welche die Zellmigration und/oder die chondrogene bzw. osteogene Differenzierung der Zellen fördern.

Die Hohlräume können mit einem Zell-Matrix-Konstrukt gefüllt werden. Ein solches Zell-Matrix-Konstrukt umfasst autologe und/oder allogene mesenchymale Stamm- und/oder Vorläuferzellen oder autologe Chondrozyten oder Periost Zellen. Die Zellen können in einer biokompatiblen Matrix, beispielsweise Kollagen, Hyaluronsäure, Alginat, Chitosan, Fibrin oder in Biopolymeren, appliziert werden. Es kann auch eine zellfreie Matrix in die Hohlräume appliziert werden. In diesem Fall können die Zellen über Verbindungen zum Knochenmarksraum, beispielsweise durch Bohrungen oder subchondrale Knochenlamellen, in die Matrix eingebunden werden.

Das Implantat kann nach einer weiteren Ausgestaltung auch mit bereits verfüllten Lücken bereitgestellt werden. Das Füllmaterial kann Zellen, ein Zell-Matrix-Konstrukt, Wachstums- und/oder Differenzierungsfaktoren und dgl. umfassen. Es können insbesondere als Füllmaterial die in den beiden vorhergehenden Absätzen genannten Zellen, Zell-Matrix-Konstrukte und dgl. verwendet werden.

Bezugszeichenliste

1   Vorsprung
2   erste Schicht
3   zweite Schicht
4   Nagel

B   Baustein
B1   erster Baustein
B2   zweiter Baustein
B3   dritter Baustein
B4   vierter Baustein
B5   fünfter Baustein
B6   sechster Baustein
B7   siebter Baustein
D   Durchbruch

| | |
|---|---|
| L | Lücke |
| O | Oberseite |
| P | Brücke |
| PS | Polymerschicht |
| S1 | erste Symmetrieachse |
| S2 | zweite Symmetrieachse |
| Ss | Seitenfläche |
| U | Unterseite |
| Z | Beschichtung |

## Patentansprüche

1. Implantat zum Ersatz von Knochen- oder Knorpelmaterial, gebildet aus einer Vielzahl von aus einem nichtmetallischen, linear elastischen Material hergestellten Bausteinen (B, B1, B2, B3, B4, B5, B6, B7), wobei ein Baustein (B, B1, B2, B3, B4, B5, B6, B7) mit benachbarten Bausteinen (B, B1, B2, B3, B4, B5, B6, B7) mittels eines viskoelastischen Polymermaterials so verbunden ist, dass zwischen den benachbarten Bausteinen (B, B1, B2, B3, B4, B5, B6, B7) Lücken (L) verbleiben und die benachbarten Bausteine (B, B1, B2, B3, B4, B5, B6, B7) relativ zueinander beweglich sind, wobei die Lücken (L) im applizierten Zustand der Aufnahme von Zellmaterial oder bioaktivem Material dienen,
   wobei das Polymermaterial aus der folgenden Gruppe ausgewählt ist: Epoxidharz, präkeramische Polymere, Silikongummi, Polylactid, Polycaprolacton, Polymethylmethaacrylate, Polylactid-co-Glycolid (PLGA), Polyhydroxyalkanoate (PHBHHX), Fibrin, Butyrate, Seide, Chitosan,
   wobei das Polymermaterial eine Polymerschicht (PS) bildet, welche die Bausteine (B, B1, B2, B3, B4, B5, B6, B7) überlagert und an die Oberseite (O) der Bausteine (B, B1, B2, B3, B4, B5, B6, B7) angebunden ist, und
   wobei die Polymerschicht (PS) Durchbrüche (D) aufweist.

2. Implantat nach Anspruch 1, wobei die Bausteine (B, B1, B2, B3, B4, B5, B6, B7) mehrere aus unterschiedlichen Materialien hergestellte Bausteine (B, B1, B2, B3, B4, B5, B6, B7) umfassen.

3. Implantat nach einem der vorhergehenden Ansprüche, wobei das linear elastische Material einen E-Modul von zumindest 10 GPa hat.

4. Implantat nach einem der vorhergehenden Ansprüche, wobei das linear elastische Material aus der folgenden Gruppe ausgewählt ist: Keramik, Glaskeramik, Glas, oder ein zumindest eines der vorgenannten Materialien enthaltender Komposit-Werkstoff.

5. Implantat nach einem der vorhergehenden Ansprüche, wobei das linear elastische Material aus der folgenden Gruppe ausgewählt ist: Aluminiumoxid, Hydroxylapatit, Beta-Tricalciumphosphat (TCP), $BaTiO_3$-Epoxidharz-Komposit, Bioglas, Bioglas-Epoxidharz-Komposite, bleifreie Epoxidharz-Komposite, bleifreie Keramiken, Lithium-, Natrium-, Kalium-Niobat, Keramik/präkeramische Polymere-Komposite, Polysiloxane, Polysilazane, Polyphosphazene, vernetzte präkeramische Polymere, gesinterte präkeramische Polymere.

6. Implantat nach einem der vorhergehenden Ansprüche, wobei zumindest ein Teil der Bausteine (B, B1, B2, B3, B4, B5, B6, B7) aus mehreren Schichten (2, 3) gebildet ist, welche aus einem unterschiedlichen Material hergestellt sind.

7. Implantat nach einem der vorhergehenden Ansprüche, wobei zumindest ein Teil der Bausteine (B, B1, B3, B4, B5, B6, B7) eine Ober- (O) und Unterseite (U) sowie die Ober- (O) mit der Unterseite (U) verbindende Seitenflächen (Ss) umfasst, wobei die Bausteine (B, B1, B3, B4, B5, B6, B7) in Draufsicht auf die Oberseite (O) einen polygonalen Umriss mit zumindest m Ecken aufweisen, wobei m eine natürliche Zahl $\geq$ 3 ist.

8. Implantat nach einem der vorhergehenden Ansprüche, wobei die Bausteine (B, B1, B3, B4) n-zählige Symmetrieachse (S1, S2) aufweisen, wobei für n gilt:

$$n = m\,/\,a,$$

wobei a eine natürliche Zahl ist.

9. Implantat nach einem der vorhergehenden Ansprüche, wobei zumindest ein Teil der Bausteine (B, B2, B5) eine ring- oder rohrartige Geometrie aufweist.

10. Implantat nach einem der vorhergehenden Ansprüche, wobei ein Übergang zwischen der Oberseite (O) und den Seitenflächen (Ss) eine Rundung aufweist.

11. Implantat nach einem der vorhergehenden Ansprüche, wobei die Ober- (O) und/oder Unterseite (U) mit einem vorgegebenen Radius gekrümmt ist.

12. Implantat nach einem der vorhergehenden Ansprüche, wobei die Oberseite (O) eine erste Rauheit und die Unterseite U eine zweite Rauheit aufweist, wobei die erste Rauheit kleiner als die zweite Rauheit ist.

13. Implantat nach einem der vorhergehenden Ansprüche, wobei an einem Außenumfang mehrere Vorsprünge (1) zum Anbinden des Polymermaterials angeformt sind.

14. Implantat nach einem der vorhergehenden Ansprüche, wobei das Polymermaterial einen E-Modul von weniger als 10 GPa hat.

15. Implantat nach einem der vorhergehenden Ansprüche, wobei die Bausteine (B, B1, B2, B3, B4, B5, B6, B7) mehrere Teilmengen umfassen, wobei Bausteine (B, B1, B2, B3, B4, B5, B6, B7) einer Teilmenge sich von den Bausteinen (B, B1, B2, B3, B4, B5, B6, B7) einer anderen Teilmenge in ihrer Geometrie unterscheiden.

16. Implantat nach einem der vorhergehenden Ansprüche, wobei die Polymerschicht (PS) eine Dicke im Bereich von 50 bis 1500 $\mu$m aufweist.

17. Implantat nach einem der vorhergehenden Ansprüche, wobei die Polymerschicht (PS) netz- oder gitterartig ausgebildet ist.

18. Implantat nach einem der vorhergehenden Ansprüche, wobei ein Baustein (B, B1, B2, B3, B4, B5, B6, B7) mit benachbarten Bausteinen (B, B1, B2, B3, B4, B5, B6, B7) über zumindest drei aus dem viskoelastischen Polymermaterial hergestellte Brücken (P) verbunden ist.

19. Implantat nach einem der vorhergehenden Ansprüche, wobei die Oberseite (O) und/oder Seitenfläche (Ss) der Bausteine (B, B1, B2, B3, B4, B5, B6, B7) mit dem Polymermaterial und/oder einem weiteren Polymermaterial beschichtet ist.

20. Implantat nach einem der vorhergehenden Ansprüche, wobei eine einzige Lage von in einer Ebene angeordneten Bausteinen (B, B1, B2, B3, B4, B5, B6, B7) mittels des Polymermaterials zu einer flexiblen Schicht verbunden sind.

21. Implantat nach einem der vorhergehenden Ansprüche, wobei mehrere aufeinander gestapelte Lagen von Bausteinen (B, B1, B2, B3, B4, B5, B6, B7) zu einer flexiblen Schicht oder einem flexiblen Block verbunden sind.

22. Kit, umfassend ein Implantat nach einem der vorhergehenden Ansprüche und Befestigungsmittel zum Befestigen des Implantats.

**Claims**

1. An implant for replacing bone or cartilage material, which is constituted by a plurality of elements (B, B1, B2, B3, B4, B5, B6, B7) produced from a non-metallic, linearly elastic material, an element (B, B1, B2, B3, B4, B5, B6, B7) being connected to adjacent elements (B, B1, B2, B3, B4, B5, B6, B7) by a viscoelastic polymer material such that gaps (L) remain between the adjacent elements (B, B1, B2, B3, B4, B5, B6, B7) and that the adjacent elements (B, B1, B2, B3, B4, B5, B6, B7) can move relative to one another, the gaps (L) being used to accommodate cell material or bioactive material in the applied state, wherein the polymer material is selected from the following group: epoxy resin, preceramic polymers, silicone rubber, polylactide, polycaprolactone, polymethylmethacrylates, polylactide-co-glycolide (PLGA), polyhydroxyalkanoates (PHBHHX), fibrin, butyrates, silk, chitosan,

wherein the polymer material forms a polymer layer (PS) which overlays the elements (B, B1, B2, B3, B4, B5, B6, B7) and is attached to the upper side (O) of the elements (B, B1, B2, B3, B4, B5, B6, B7), and

wherein the polymer layer (PS) has apertures (D).

2. The implant according to claim 1, wherein the elements (B, B1, B2, B3, B4, B5, B6, B7) comprise a plurality of elements (B, B1, B2, B3, B4, B5, B6, B7) produced from different materials.

3. The implant according to either of the preceding claims, wherein the linearly elastic material has a modulus of elasticity of at least 10 GPa.

4. The implant according to any one of the preceding claims, wherein the linearly elastic material is selected from the following group: ceramic, glass ceramic, glass, or a composite material containing at least one of the aforementioned materials.

5. The implant according to any one of the preceding claims, wherein the linearly elastic material is selected from the following group: aluminium oxide, hydroxyapatite, beta-tricalcium phosphate (TCP), $BaTiO_3$ epoxy resin composite, bioglass, bioglass epoxy resin composites, lead-free epoxy resin composites, lead-free ceramics, lithium, sodium, potassium niobate, ceramic/preceramic polymer composites, polysiloxanes, polysilazanes, polyphosphazenes, crosslinked preceramic polymers, and sintered preceramic polymers.

6. The implant according to any one of the preceding claims, wherein at least some of the elements (B, B1, B2, B3, B4, B5, B6, B7) are produced from a plurality of layers (2, 3) made of a different material.

7. The implant according to any one of the preceding claims, wherein at least some of the elements (B, B1, B3, B4, B5, B6, B7) comprise a upper side (O) and lower side (U) as well as side faces (Ss) connecting the upper side (O) to the lower side (U), the elements (B, B1, B3, B4, B5, B6, B7) having a polygonal outline with at least m corners in plan view of the upper side (O), where m is a natural number $\geq 3$.

8. The implant according to any one of the preceding claims, wherein the elements (B, B1, B3, B4) have an n-fold axis of symmetry (S1, S2), with the following being true for n:

$$n = m \,/\, a,$$

where a is a natural number.

9. The implant according to any one of the preceding claims, wherein at least some of the elements (B, B2, B5) have an annular or tubular geometry.

10. The implant according to any one of the previous claims, wherein a transition between the upper side (O) and the side faces (Ss) has a rounded shape.

11. The implant according to any one of the preceding claims, wherein the upper side (O) and/or lower side (U) is curved with a predefined radius.

12. The implant according to any one of the preceding claims, wherein the upper side (O) has a first roughness and the lower side U has a second roughness, the first roughness being smaller than the second roughness.

13. The implant according to any one of the preceding claims, wherein a plurality of projections (1) for attaching the polymer material are formed on an outer circumference.

14. The implant according to any one of the preceding claims, wherein the polymer material has a modulus of elasticity of less than 10 GPa.

15. The implant according to any one of the preceding claims, wherein the elements (B, B1, B2, B3, B4, B5, B6, B7) comprise a plurality of subsets, with elements (B, B1, B2, B3, B4, B5, B6, B7) of one subset differing from the elements (B, B1, B2, B3, B4, B5, B6, B7) of another subset in respect of their geometry.

16. The implant according to any one of the preceding claims, wherein the polymer layer (PS) has a thickness in the range of 50 to 1500 μm.

17. The implant according to any one of the preceding claims, wherein the polymer layer (PS) is reticular or lattice-like.

18. The implant according to any one of the preceding claims, wherein an element (B, B1, B2, B3, B4, B5, B6, B7) is connected to adjacent elements (B, B1, B2, B3, B4, B5, B6, B7) by at least three bridges (P) made of the viscoelastic polymer material.

19. The implant according to any one of the preceding claims, wherein the upper side (O) and/or side face (Ss) of the elements (B, B1, B2, B3, B4, B5, B6, B7) is coated with the polymer material and/or a further polymer material.

20. The implant according to any one of the preceding claims, wherein a single layer of elements (B, B1, B2, B3, B4, B5, B6, B7) arranged in one plane are joined together by means of the polymer material to form a flexible layer.

21. The implant according to any one of the preceding claims, wherein a plurality of stacked layers of elements (B, B1, B2, B3, B4, B5, B6, B7) are connected to form a flexible layer or a flexible block.

22. A kit comprising an implant according to any one of the preceding claims and fastening means for fastening the implant.

**Revendications**

1. Implant destiné à remplacer un matériau osseux ou cartilagineux, formé d'une pluralité de modules (B, B1, B2, B3, B4, B5, B6, B7) fabriqués à partir d'un matériau non métallique, élastique linéairement, en ce qu'un module (B, B1, B2, B3, B4, B5, B6, B7) est raccordé à des modules voisins (B, B1, B2, B3, B4, B5, B6, B7) au moyen d'un matériau polymère viscoélastique de façon à laisser des interstices (L) entre les modules voisins (B, B1, B2, B3, B4, B5, B6, B7) et à permettre aux modules voisins (B, B1, B2, B3, B4, B5, B6, B7) d'être mobiles les uns par rapport aux autres, en ce que les interstices (L) à l'état appliqué servent à la réception de matériau cellulaire ou matériau bioactif, en ce que le matériau polymère est sélectionné parmi le groupe suivant :
   résine époxyde, polymères précéramiques, caoutchouc de silicone, polylactide, polycaprolactone, polyméthacrylate de méthyle, polylactide-co-glycolide (PLGA), polyhydroxyalcanoates (PHBHHX), fibrine, butyrate, soie, chitosane, en ce que le matériau polymère forme une couche de polymère (PS) qui superpose les modules (B, B1, B2, B3, B4, B5, B6, B7) et est reliée à la face supérieure (O) des modules (B, B1, B2, B3, B4, B5, B6, B7),et en ce que la couche de polymère (PS) présente des passages (D).

2. Implant selon la revendication 1, en ce que les modules (B, B1, B2, B3, B4, B5, B6, B7) comportent plusieurs modules (B, B1, B2, B3, B4, B5, B6, B7) fabriqués à partir de matériaux différents.

3. Implant selon l'une des revendications précédentes, en ce que le matériau élastique linéairement a un module d'élasticité E d'au moins 10 GPa.

4. Implant selon l'une des revendications précédentes, en ce que le matériau élastique linéairement est sélectionné parmi le groupe suivant : céramique, vitrocéramique, verre ou un matériau composite contenant au moins l'un des matériaux précités.

5. Implant selon l'une des revendications précédentes, en ce que le matériau élastique linéairement est sélectionné parmi le groupe suivant : oxyde d'aluminium, hydroxyapatite, phosphate tricalcique bêta (TCP), composite résine époxyde BaTiO$_3$, bioverre, composites résine époxyde-bioverre, composites résine époxyde sans plomb, céramiques sans plomb, composites polymères céramiques/précéramiques à base de lithium, sodium, niobate de potassium, polysiloxane, polysilazane, polyphosphazène, polymères précéramiques réticulés, polymères précéramiques frittés.

6. Implant selon l'une des revendications précédentes, en ce qu'au moins une partie des modules (B, B1, B2, B3, B4, B5, B6, B7) est formée de plusieurs couches (2, 3), lesquelles sont fabriquées à partir d'un matériau différent.

**7.** Implant selon l'une des revendications précédentes, en ce qu'au moins une partie des modules (B, B1, B3, B4, B5, B6, B7) comporte une face supérieure (O) et une face inférieure (U) ainsi que des faces latérales (Ss) reliant la face supérieure (O) à la face inférieure (U), en ce que les modules (B, B1, B3, B4, B5, B6, B7) vue de dessus sur la face supérieure (O) présentent un contour polygonal avec au moins m angles, m étant un entier naturel $\geq 3$.

**8.** Implant selon l'une des revendications précédentes, en ce que les modules (B, B1, B3, B4) présentent un axe de symétrie d'ordre n (S1, S2), en ce que n :

$$n = m / a,$$

a étant un entier naturel.

**9.** Implant selon l'une des revendications précédentes, en ce qu'au moins une partie des modules (B, B2, B5) présente une géométrie annulaire ou tubulaire.

**10.** Implant selon l'une des revendications précédentes, en ce qu'une transition entre la face supérieure (O) et les faces latérales (Ss) présente un arrondi.

**11.** Implant selon l'une des revendications précédentes, en ce que la face supérieure (O) et/ou la face inférieure (U) à un rayon prédéfini est courbe.

**12.** Implant selon l'une des revendications précédentes, en ce que la face supérieure (O) présente une première rugosité et la face inférieure (U) une seconde rugosité, en ce que la première rugosité est inférieure à la seconde rugosité.

**13.** Implant selon l'une des revendications précédentes, en ce que plusieurs parties en saillie (1) sont formées sur une circonférence extérieure pour la liaison du matériau polymère.

**14.** Implant selon l'une des revendications précédentes, en ce que le matériau polymère a un module d'élasticité E inférieur à 10 GPa.

**15.** Implant selon l'une des revendications précédentes, en ce que les modules (B, B1, B2, B3, B4, B5, B6, B7) comportent plusieurs sous-ensembles, en ce que des modules (B, B1, B2, B3, B4, B5, B6, B7) d'un sous-ensemble diffèrent des modules (B, B1, B2, B3, B4, B5, B6, B7) d'un autre sous-ensemble dans leur géométrie.

**16.** Implant selon l'une des revendications précédentes, en ce que la couche de polymère (PS) présente une épaisseur dans une plage comprise entre 50 et 1500 $\mu$m.

**17.** Implant selon l'une des revendications précédentes, en ce que la couche de polymère (PS) est réalisée de manière réticulée ou frittée.

**18.** Implant selon l'une des revendications précédentes, en ce qu'un module (B, B1, B2, B3, B4, B5, B6, B7) est relié à des modules voisins (B, B1, B2, B3, B4, B5, B6, B7) par l'intermédiaire d'au moins trois ponts (P) réalisés à partir du matériau polymère viscoélastique.

**19.** Implant selon l'une des revendications précédentes, en ce que la face supérieure (O) et/ou la face latérale (Ss) des modules (B, B1, B2, B3, B4, B5, B6, B7) est revêtue du matériau polymère et/ou d'un autre matériau polymère.

**20.** Implant selon l'une des revendications précédentes, en ce qu'une seule couche de modules (B, B1, B2, B3, B4, B5, B6, B7) disposés dans un plan est reliée en une couche flexible au moyen du matériau polymère.

**21.** Implant selon l'une des revendications précédentes, en ce que plusieurs couches empilées l'une sur l'autre de modules (B, B1, B2, B3, B4, B5, B6, B7) sont reliées en une couche flexible ou un bloc flexible.

**22.** Kit comprenant un implant selon l'une des revendications précédentes et des moyens de fixation pour la fixation de l'implant.

# Fig. 1a

# Fig. 1b

# Fig. 2a

# Fig. 2b

# Fig. 3a

B3

# Fig. 3b

S1

# Fig. 4a

B4

O

U

# Fig. 4b

O

S2

**Fig. 5**

B5

A

1

O

**Fig. 6**

B6

O

3

2

Ss

U

**Fig. 6a**

Z   O   L   PS   O   Z

B   U   Ss   Ss   U   B

# Fig. 7

# Fig. 8

**Fig. 9**

**Fig. 10**

**Fig. 11**

**Fig. 12**

**Fig. 13**

**Fig. 13a**

**Fig. 14**

## Fig. 15a

## Fig. 15b

## Fig. 15c

## Fig. 16

## Fig. 17

## Fig. 18

B7

O

Ss

**EP 3 672 533 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2014006519 A1
- DE 10303660 B4
- EP 0144209 B1
- EP 0197441 B1
- DE 10022260 C2
- DE 10157315 C1
- EP 1646334 B1
- EP 1442726 B1
- EP 2104471 B1
- EP 2296583 B1
- DE 102016211201 A1
- EP 0654250 A
- EP 1712205 A2